(19) Europäisches Patentamt
European Patent Office
Office européen des brevets

(11) **EP 2 177 608 B1**

(12) **EUROPEAN PATENT SPECIFICATION**

(45) Date of publication and mention
of the grant of the patent:
**14.10.2015 Bulletin 2015/42**

(21) Application number: **08791744.9**

(22) Date of filing: **28.07.2008**

(51) Int Cl.:
*C12N 11/14* [(2006.01)]    *C12N 9/20* [(2006.01)]
*C12N 11/02* [(2006.01)]

(86) International application number:
**PCT/JP2008/063509**

(87) International publication number:
**WO 2009/017087 (05.02.2009 Gazette 2009/06)**

(54) **IMMOBILIZED LIPASE AND METHOD FOR PRODUCING THE SAME**

IMMOBILISIERTE LIPASE UND HERSTELLUNGSVERFAHREN DAFÜR

LIPASE IMMOBILISÉE ET SON PROCÉDÉ DE FABRICATION

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **31.07.2007 JP 2007199240**

(43) Date of publication of application:
**21.04.2010 Bulletin 2010/16**

(73) Proprietor: **Fuji Oil Company, Limited
Izumisano-shi
Osaka 598-8540 (JP)**

(72) Inventors:
• **IWAOKA, Eiji
Izumisano-shi
Osaka 598-8540 (JP)**
• **ARISHIMA, Toshiharu
Tsukubamirai-shi
Ibaraki 300-2436 (JP)**

(74) Representative: **Boult Wade Tennant
Verulam Gardens
70 Gray's Inn Road
London WC1X 8BT (GB)**

(56) References cited:
WO-A1-2004/035773    JP-A- 02 138 986
JP-A- 11 500 319     JP-A- 62 134 090
JP-A- 2001 506 499   US-A- 5 177 013
US-A1- 2007 270 507

• BUTHE A. ET AL.: 'Generation of lipase-containing static emulsions in silicone spheres for synthesis in organic media' J. MOL. CATAL. B: ENZYMATIC vol. 35, no. 4-6, 2005, pages 93 - 99, XP005027013
• LAMB S.B. ET AL.: 'Enzyme immobilisation on colloidal liquid aphrons (CLAs): influence of protein properties' ENZYME MICROB. TECHNOL. vol. 24, 1999, pages 541 - 548, XP008129481
• SCHULZ P C ET AL: "Emulsification properties of chitosan", COLLOID AND POLYMER SCIENCE 1998 DE LNKD- DOI:10.1007/S003960050359, vol. 276, no. 12, 1998, pages 1159-1165, ISSN: 0303-402X

**Description**

Technical Field

The present invention relates to a highly active immobilized lipase suitably used for, for example, various esterification reactions and transesterification reactions, and a method for producing the same.

Background Art

Modification of a fat/oil with a transesterification reaction using a lipase as a catalyst has widely been carried out. There have been known a batch method, a continuous method with a column, and the like as the method of transesterification reaction of a fat/oil. Traditionally, the lipase has generally been used as an immobilized lipase obtained by immobilizing the lipase to a certain carrier. For the lipase immobilization, an anion exchange resin (Patent Document 1), a phenol formaldehyde absorbing resin (Patent Document 2), a hydrophobic porous carrier (Patent Document 3), a cation exchange resin (Patent Document 4), a chelate resin (Patent Document 5), and the like have been used, but the methods disclosed in these Documents have the drawback of a reduction in transesterification activity due to the immobilization of the lipase to the carrier as well as the tendency that the cost is increased due to the expensive carrier. Also, as a method for enhancing activity during immobilization, addition of a polyhydroxy compound that does not have emulsification activity (Patent Document 6) and addition of a fatty acid derivative (Patent Document 7) have been proposed, but these methods are not satisfactory yet.

> Patent Document 1: JP 60-98984 A
> Patent Document 2: JP 61-202688 A
> Patent Document 3: JP 2-138986 A
> Patent Document 4: JP 3-61485 A
> Patent Document 5: JP 1-262795 A
> Patent Document 6: JP 7-265073 A
> Patent Document 7: JP 62-134090 A

WO 2004/035773 relates to a process for immobilizing a compound containing a specified functional group onto an insoluble polymer matrix.
US 5,177,013 relates to a process of preparing an immobilized lipase preparation useful for modification of fats and oils.

Disclosure of Invention

Problems to be Solved by the Invention

An object of the present invention is to provide an immobilized lipase, transesterification activity of which is improved as compared to conventional methods.

Means for Solving the Problems

The present inventors have intensively studied to solve the above problems and, as a result, have found that an immobilized lipase obtained by immobilizing an oil-in-water emulsion containing a fat/oil, a polymer emulsifier, and an enzyme having lipase activity to a carrier is prominently improved in transesterification activity. The present invention has been completed on the basis of this finding.
That is, the present invention relates to:

(1) an immobilized lipase composition, comprising an oil-in-water emulsion which is immobilized to a carrier, wherein the oil-in-water emulsion contains a triglyceride, a polymer emulsifier and an enzyme having lipase activity wherein the polymer emulsifier is a polysaccharide, and wherein the composiiton has improved transesterification activity compared to conventional methods of immobilization;
(2) the immobilized lipase according to (1), wherein the polymer emulsifier is a gum arabic;
(3) the immobilized lipase according to (1), wherein the polymer emulsifier is a water-soluble soybean polysaccharide;
(4) a method for producing an immobilized lipase composition, with improved transesterification activity compared to conventional methods of immobilization comprising: preparing an oil-in-water emulsion containing a triglyceride, a polymer emulsifier, and an enzyme having lipase activity; absorbing the oil-in-water emulsion to a carrier, followed by drying, wherein the polymer emulsifier is a polysaccharide.

Effect of the Invention

According to the present invention, since transesterification activity of immobilized lipase can be improved, it is possible to increase a transesterification reaction amount per unit of enzyme weight as well as to improve productivity of transesterified oil.

Best Mode for Carrying out the Invention

Hereinafter, the present invention will be described specifically. A polymer emulsifier to be used in the present invention is a polymer having emulsifying capability and is preferably a natural polymer emulsifier, and examples thereof include animal-derived protein, plant-derived protein, and emulsifying polysaccharide. More specifically, the animal-derived protein includes , for example, protein material such as skimmed milk powder and whole milk powder derived from various animals; protein such as casein, lacto-albumin, and gelatin obtained by fractionating and purifying of the protein material; and hydrolysate thereof. The plant-derived protein includes protein material such as, for example, isolated protein derived from beans such as soybean, pea, red bean, and broad bean; protein such as glycinin, β-conglycinin, and phaseolin obtained by fractionating the protein material; and hydrolysate thereof. The emulsifying polysaccharide includes various polysaccharides having emulsifying activity, such as, for example, water-soluble soybean polysaccharide, gum arabic, and various modified starches. Among the above, it is preferable to use the emulsifying polysaccharide, particularly the water-soluble soybean polysaccharide, as the emulsifier because transesterification activity is strongly maintained. In the present invention, the above-described polymer emulsifier is the essential component, but it is also possible to use a low molecular emulsifier in combination with the polymer emulsifier.

As a fat/oil to be used for preparation of an oil-in-water emulsion, it is possible to use, for example, animal and plant fat/oils such as soybean oil, coconut oil, palm oil, palm kernel oil, rapeseed oil, sunflower oil, peanut oil, olive oil, rice oil, shea butter, sal fat, cotton seed oil, cacao butter, olive oil, sesame seed oil, wheat germ oil, illipe butter, safflower oil, corn oil, milk fat, mutton fat, goat fat, horse fat, egg yolk oil, sardine oil, whale oil; triglycerides including fractionated or hydrogenated substances of the above-listed animal and plant fat/oils; lower alcohol ester of fatty acid; glycerin fatty acid monoester, glycerin fatty acid diester; or combination thereof.

In the preparation of the oil-in-water emulsion, an amount of the polymer emulsifier with respect to the fat/oil is not particularly limited insofar as stable emulsification is performed. If a desirable range is set, the amount of the polymer emulsifier to be mixed with the fat/oil is preferably 0.1 to 50 wt%, more preferably 1 to 40 wt%, most preferably 5 to 30 wt%. When the ratio between the polymer emulsifier and the fat/oil is within the above-specified range, a stable oil-in-water emulsion is obtained, thereby giving high transesterification activity after immobilization.

A mixture liquid containing the polymer emulsifier and the fat/oil is formed into the oil-in-water emulsion with an appropriate emulsifying treatment. The emulsification can be carried out by using, for example, a homogenizer, Homomixer, or Nanomizer, at appropriate pressure or by using membrane emulsification or ultrasonic wave. An average particle diameter of the oil-in-water emulsion to be used is preferably 10 μm or less, and more preferably 3 μm or less. In addition, since preparation the oil-in-water emulsion having an average particle diameter of 0.5 μm or less makes poor industrial production efficiency, the use of the oil-in-water emulsion having particle diameter larger than 0.5 μm is the common practice.

As a lipase to be used in the present invention, those of wide range of origins derived from, for example, bacteria, yeasts, filamentous bacteria, and actinomycetes can be used without particular limitation, and specific example thereof includes genus *Rhizopus* (*Rhizopus sp.*), genus *Aspergillus* (*Aspergillus sp.*), genus *Candida* (*Candida sp.*), genus *Mucor* (*Mucor sp.*), genus *Psudomonas* (*Psudomonas sp.*), genus *Alcaligenes* (*Alcaligenes sp.*), genus *Arthrobacter* (*Arthrobacter sp.*), genus *Staphylococcus* (*Staphylococcus* sp.), genus *Penicillum* (*Penicillum sp.*), and genus *Geotrichum* (*Geotrichum sp.*). Also, animal-derived and plant-derived lipase such as pancreatic lipase and rice bran lipase can be used. A crude enzyme or purified enzyme of any of these lipases is mixed as an enzyme having lipase activity with the oil-in-water emulsion that has been prepared in advance. Alternatively, the oil-in-water emulsion can also be obtained by subjecting a mixture liquid containing the polymer emulsifier, the fat/oil, and the lipase to an emulsifying treatment. The use of the lipase having high heat resistance allows the use of the oil-in-water emulsion containing a high melting point fat/oil, thereby an immobilized enzyme highly resistant to flavor deterioration caused by oxidation or hydrolysis of fat/oil can be obtained.

A mixing ratio between the enzyme and the emulsion can be varied as a ratio of the fat/oil component in the emulsion to the lipase activity of the enzyme. More specifically, a ratio of the fat/oil component amount (mg) with respect to lipase activity (Unit) measured in accordance with "JIS K0601: Industrial Lipase Activity Measurement Method" is preferably 0.06 or more, more preferably 0.4 or more. Also, the ratio is preferably 10 or less, and more preferably 3 or less. When the ratio is less than 0.06, the effect is not sufficiently exhibited in some cases. When the ratio is larger than 10, the activity per unit weight of the immobilized lipase to be prepared is decreased, and the amount of the immobilized lipase to be added to the reaction system is increased, and the reaction system is enlarged, or a moisture content derived from the carrier is increased, thereby easily causing a side reaction. The mixing of the enzyme with the emulsion can be carried out by adding the enzyme in the form of a liquid to the oil-in-water emulsion followed by homogenizing or by adding the enzyme in the form of solid or powder into the oil-in-water emulsion followed by dissolving or dispersing. Alternatively, the oil-in-water emulsion can also be obtained by subjecting the mixture liquid containing the polymer emulsifier, the fat/oil, and the enzyme to an emulsifying treatment.

The carrier to be used in the present invention includes, for example, kieselguhr, alumina, Celite, cellulose and cellulose derivatives, porous glass, glass fiber, silicic acid gel, florisil, ion exchange resin, titanium dioxide, kaolinite, and pearlite, but an ingredient of the carrier is not particularly limited insofar as the carrier, has the function of capturing lipase molecules.

Preparation of the immobilized lipase is carried out by contacting the oil-in-water emulsion containing above-described lipase with above-described carrier, followed by drying, thereby the lipase is absorbed to the carrier. For contacting, the carrier can be soaked into the oil-in-water emulsion containing the lipase, or the emulsion can be sprayed to the carrier. In the case of contacting the oil-in-water emulsion to the carrier, a moisture content that can be absorbed by the carrier is preferred. When the contact is carried out in the moisture content larger than the absorbable amount, it is difficult for the carrier to carry the predetermined enzyme amount due to leakage of the oil-in-water emulsion from the carrier.

The carrier which is absorbing the lipase can be dried by various drying methods. More specifically, the drying method includes, for example, drying means by reducing a pressure; and so-called through-flow drying in which the carrier is brought into contact with a relatively low humidity air, nitrogen, or another inert gas.

In particular, drying under reduced pressure, in which the carrier to which the lipase is absorbed is retained under a pressure environment that is lower than a water vapor pressure at a certain temperature, is preferred.

Since the immobilized lipase prepared as described above has high transesterification activity, the immobilized lipase is suitably used for, for example, modification of fat/oil. An embodiment of the immobilized lipase includes the use in various methods such as batch method and continuous method using a column.

Examples

Examples will be described in the following, however the technical scope of the present invention is not limited to them.

(Example 1)

An oil-in-water emulsion A was prepared by: dispersing and dissolving 8 g of water-soluble soybean polysaccharide into 60 g of water; adding 32g of purified palm oil to the solution; preliminary emulsifying with a Homomixer (manufactured by Tokushu Kika Kogyo Co., Ltd); and emulsifying by passing the preliminary emulsion through a high pressure laboratory homogenizer (manufactured by Ranni a/s) twice at 150 kg/cm$^2$. The obtained emulsion had an average particle diameter of 1.8 $\mu$m. A hydrate was obtained by dispersing 7 g of commercially available lipase powder manufactured by Amano Enzyme Inc. (originating from Penicillium; hydrolysis activity: 1,000 unit/g) into 10 g of cold water; mixing the dispersion with 35 g of the oil-in-water emulsion A; and spraying the mixture onto 50 g of kieselguhr. The kieselguhr was freeze-dried to obtain an immobilized lipase 1 having a moisture content of 3 wt%.

Transesterification activity was determined by adding 10 g of the immobilized lipase 1 to 100 g of a substrate (substrate moisture content: < 0.1%) obtained by blending a palm middle melting point fraction and a palm low melting point fraction at a ratio of 6:4, and then activating at 60°C. In an evaluation of a reaction product, tripalmitin content was measured with a gas chromatography (GC), and reaction rate was calculated by the following formula.

```
* Reaction rate = (tripalmitin content of reaction product

- tripalmitin content of starting fat or oil)/(tripalmitin

content of composition in reaction equilibrium state -

tripalmitin content of starting fat or oil)
```

Next, an initial reaction velocity constant k was calculated by the following formula.

* Initial reaction velocity constant k = ln[1/(1 − reaction rate after 24 hours reaction)]

A value calculated from the reaction velocity constant k and the amount of the lipase (amount of enzyme preparation having lipase activity contained in immobilized lipase) added to the starting fat/oil with the following formula was used as a transesterification activity value, and the value is shown in Table 1.

* Transesterification activity value = k × (amount of starting fat or oil/amount of enzyme preparation having lipase activity)

(Example 2)

A hydrate was obtained by dispersing 7 g of commercially available lipase powder manufactured by Amano Enzyme Inc. (same as above) into 10 g of cold water; mixing the dispersion with 17.5 g of the oil-in-water emulsion A; and dispersing 50 g of kieselguhr into the mixture. After that, preparation and evaluation of immobilized lipase were carried out in the same manner as in Example 1.

(Example 3)

Preparation and evaluation of immobilized lipase were carried out in the same manner as in Example 2, except that the added amounts of glucose and the oil-in-water emulsion A were 13.3 g and 1.75 g respectively.

(Example 4)

Preparation and evaluation of immobilized lipase were carried out in the same manner as in Example 2, except that the added amounts of glucose and the oil-in-water emulsion A were 13.7 g and 0.88 g respectively.

(Example 5)

An oil-in-water emulsion B was prepared according to the preparation method in Example 1, except that the pressure of the high pressure laboratory homogenizer was 50 kg/cm$^2$. The obtained emulsion had an average particle diameter of 4.8 $\mu$m. Preparation and evaluation of immobilized lipase were carried out in the same manner as in Example 1 except for using the oil-in-water emulsion B.

(Example 6)

An oil-in-water emulsion C was prepared according to the preparation method in Example 1, except that a gum arabic was used in place of the water-soluble soybean polysaccharide. The obtained emulsion had an average particle diameter of 2 $\mu$m. Preparation and evaluation of immobilized lipase were carried out in the same manner as in Example 1 except for using the oil-in-water emulsion C.

(Example 7)

An oil-in-water emulsion D was prepared according to the preparation method in Example 1, except that commercially available casein sodium (EMLV: manufactured by DMV) was used in place of the water-soluble soybean polysaccharide. The obtained emulsion had an average particle diameter of 2.2 $\mu$m. Preparation and evaluation of immobilized lipase were carried out in the same manner as in Example 1 except for using the oil-in-water emulsion D.

(Example 8)

An oil-in-water emulsion E was prepared according to the preparation method in Example 1, except that an isolated soybean protein (Fujipuro R: manufacutured by Fuji Oil Co., Ltd.) was used in place of the water-soluble soybean polysaccharide. The obtained emulsion had an average particle diameter of 3.5 μm. Preparation and evaluation of immobilized lipase were carried out in the same manner as in Example 1 except for using the oil-in-water emulsion E.

(Comparative Example 1)

An oil-in-water emulsion F was prepared according to the preparation method in Example 1, except that commercially available sucrose fatty acid ester (DK-SS: manufactured by Dai-ichi Kogyo Seiyaku Co., Ltd.) was used in place of the water-soluble soybean polysaccharide. The obtained emulsion had an average particle diameter of 2.4 μm. Preparation and evaluation of immobilized lipase were carried out in the same manner as in Example 1 except for using the oil-in-water emulsion F.

(Comparative Examples 2 to 4)

Evaluations were carried out in the same manner as in Comparative Example 1 except for using sucrose fatty acid ester (DK-F-160: manufactured by Dai-ichi Kogyo Seiyaku Co., Ltd./Comparative Example 2), polyglycerin fatty acid ester (Sansoft Q14S: manufactured by Taiyo Kagaku Co., Ltd./Comparative Example 3), and polyglycerin fatty acid ester (Sansoft Q12S: manufactured by Taiyo Kagaku Co., Ltd./Comparative Example 4) in place of the sucrose fatty acid ester (DK-SS).

(Comparative Example 5)

Preparation and evaluation of immobilized lipase were carried out in the same manner as in Example 1 except for using 14 g of glucose and 21 g of water in place of 35 g of the oil-in-water emulsion A.

(Table 1) Reactivity and Transesterification Activity after 24 hours Reaction

| | Composition | | | | | | Enzyme activity | |
|---|---|---|---|---|---|---|---|---|
| | Fat/oil (g) | Emulsifier (g) | Glucose (g) | Enzyme | | Fat/oil (mg)/Enzyme Units | Reaction rate (%) | Transesterification activity |
| | | | | (g) | (Unit) | | | |
| Example 1 | 11.2 | 2.8 | - | 7.0 | 7,000 | 1.6 | 80 | 161 |
| Example 2 | 5.6 | 1.4 | 7.0 | ↑ | ↑ | 0.80 | 75 | 139 |
| Example 3 | 0.56 | 0.14 | 13.3 | ↑ | ↑ | 0.08 | 45 | 60.0 |
| Example 4 | 0.28 | 0.07 | 13.7 | ↑ | ↑ | 0.04 | 28 | 33.0 |

(Table 2) Reactivity and Transesterification Activity after 24 hours Reaction

| | Emulsifier | Composition | | | | | | Emulsion state | | Enzyme activity | |
|---|---|---|---|---|---|---|---|---|---|---|---|
| | | Fat/oil (g) | Emulsifier (g) | Glucose (g) | Enzyme | | Fat/oil (mg)/Enzyme Units | Homogenizing pressure (kgf) | Emulsion particle diameter (μm) | Reaction rate (%) | Transesterification activity |
| | | | | | (g) | (Unit) | | | | | |
| Example 1 | Water-soluble soybean polysaccharide | 11.2 | 2.8 | - | 7.0 | 7,000 | 1.6 | 150 | 1.8 | 80 | 161 |
| Example 5 | ↑ | ↑ | ↑ | - | ↑ | ↑ | ↑ | 50 | 4.8 | 70 | 120 |
| Example 6 | Gum arabic | ↑ | ↑ | - | ↑ | ↑ | ↑ | 150 | 2.0 | 72 | 127 |
| Example 7 | Casein Na | ↑ | ↑ | - | ↑ | ↑ | ↑ | ↑ | 2.2 | 58 | 86.8 |
| Example 8 | Soybean protein | ↑ | ↑ | - | ↑ | ↑ | ↑ | ↑ | 3.5 | 63 | 99.4 |
| Comparative Example 1 | Sucrose ester | ↑ | ↑ | - | ↑ | ↑ | ↑ | ↑ | 2.4 | 45 | 59.8 |
| Comparative Example 2 | Sucrose ester | ↑ | ↑ | - | ↑ | ↑ | ↑ | ↑ | 1.6 | 41 | 52.8 |
| Comparative Example 3 | Polyglycerin ester | ↑ | ↑ | - | | ↑ | ↑ | ↑ | 2.2 | 42 | 54.5 |
| Comparative Example 4 | Polyglycerin ester | ↑ | ↑ | - | ↑ | ↑ | ↑ | ↑ | 3.7 | 39 | 49.4 |
| Comparative Example 5 | - | - | - | 14 | ↑ | ↑ | - | - | - | 15 | 19.8 |

EP 2 177 608 B1

From the results shown in Table 1, it was confirmed that the transesterification activity was increased by adding the emulsion in an amount that the solid component (mg) of the emulsion was 0.05 times or more of the lipase activity (Unit), and that the trans-esterification activity can be prominently enhanced by adding the emulsion in an amount that the solid component (mg) of the emulsion was 0.1 times or more of the lipase activity (Unit), as compared to the products without adding the emulsion (Comparative Example 2). As the emulsifier, the polysaccharide having emulsifying property was particularly preferable. It was also confirmed that the effect was tend to reduce when the average particle diameter of the oil-in-water emulsion to be added was larger than 4 μm (Example 5).

## Claims

1. An immobilized lipase composition, comprising an oil-in-water emulsion which is immobilized to a carrier, wherein the oil-in-water emulsion contains a triglyceride, a polymer emulsifier and an enzyme having lipase activity, wherein the polymer emulsifier is a polysaccharide, wherein the composition has improved transesterification activity compared to conventional methods of immobilization.

2. The immobilized lipase composition according to claim 1, wherein the polymer emulsifier is gum arabic.

3. The immobilized lipase composition according to claim 1, wherein the polymer emulsifier is a water-soluble soybean polysaccharide.

4. A method for producing an immobilized lipase composition with improved transesterification activity compared to conventional methods of immobilization, comprising: preparing an oil-in-water emulsion containing a triglyceride, a polymer emulsifier, and an enzyme having lipase activity; absorbing the oil-in-water emulsion to a carrier, followed by drying, wherein the polymer emulsifier is a polysaccharide.

## Patentansprüche

1. Immobilisierte Lipasezusammensetzung, umfassend eine Öl-in-Wasser-Emulsion, die an einen Träger immobilisiert ist, worin die Öl-in-Wasser-Emulsion ein Triglycerid, einen Polymeremulgator und ein Enzym mit Lipaseaktivität enthält, worin der Polymeremulgator ein Polysaccharid ist, worin die Zusammensetzung eine verbesserte Umesterungsaktivität im Vergleich zu konventionellen Verfahren der Immobilisierung hat.

2. Immobilisierte Lipasezusammensetzung nach Anspruch 1, worin der Polymeremulgator Gummi arabicum ist.

3. Immobilisierte Lipasezusammensetzung nach Anspruch 1, worin der Polymeremulgator ein wasserlösliches Sojabohnenpolysaccharid ist.

4. Verfahren zur Erzeugung einer immobilisierten Lipasezusammensetzung mit verbesserter Umesterungsaktivität im Vergleich zu konventionellen Verfahren der Immobilisierung, umfassend: Herstellung einer Öl-in-Wasser-Emulsion, umfassend ein Triglycerid, einen Polymeremulgator und ein Enzym mit Lipaseaktivität, Absorption der Öl-in-Wasser-Emulsion an einen Träger, mit anschließendem Trocknen, worin der Polymeremulgator ein Polysaccharid ist.

## Revendications

1. Composition de lipase immobilisée, comprenant une émulsion huile dans l'eau qui est immobilisée sur un support, dans laquelle l'émulsion huile dans l'eau contient un triglycéride, un émulsionnant polymère et une enzyme ayant une activité de lipase, dans laquelle l'émulsionnant polymère est un polysaccharide, laquelle composition a une meilleure activité de trans-estérification qu'avec les procédés conventionnels d'immobilisation.

2. Composition de lipase immobilisée selon la revendication 1, dans laquelle l'émulsionnant polymère est la gomme arabique.

3. Composition de lipase immobilisée selon la revendication 1, dans laquelle l'émulsionnant polymère est un polysaccharide de soja soluble dans l'eau.

4. Procédé pour produire une composition de lipase immobilisée ayant une meilleure activité de trans-estérification qu'avec les procédés conventionnels d'immobilisation, comprenant : la préparation d'une émulsion huile dans l'eau contenant un triglycéride, un émulsionnant polymère, et une enzyme ayant une activité de lipase ; l'absorption de l'émulsion huile dans l'eau sur un support, suivie d'un séchage, dans lequel l'émulsionnant polymère est un poly-saccharide.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 60098984 A
- JP 61202688 A
- JP 2138986 A
- JP 3061485 A
- JP 1262795 A
- JP 7265073 A
- JP 62134090 A
- WO 2004035773 A
- US 5177013 A